Europäisches Patentamt

European Patent Office

Office européen des brevets

(19)

(11) Publication number: **0 081 948**
**B1**

(12)                    **EUROPEAN PATENT SPECIFICATION**

(45) Date of publication of patent specification: **13.05.87**

(51) Int. Cl.⁴: **B 01 J 8/02, C 07 C 29/15**

(21) Application number: **82306406.8**

(22) Date of filing: **02.12.82**

(54) **Reactor.**

(30) Priority: **14.12.81 GB 8137658**

(43) Date of publication of application:
**22.06.83 Bulletin 83/25**

(45) Publication of the grant of the patent:
**13.05.87 Bulletin 87/20**

(84) Designated Contracting States:
**DE FR GB IT NL**

(56) References cited:
**DE-C- 719 652**
**GB-A-1 073 118**
**GB-A-1 205 156**
**GB-A-2 046 618**
**US-A-2 961 304**

(73) Proprietor: **IMPERIAL CHEMICAL INDUSTRIES PLC**
**Imperial Chemical House Millbank**
**London SW1P 3JF (GB)**

(72) Inventor: **Pinto, Alwyn**
**18 Cambridge Road**
**Linthorpe Middlesbrough Cleveland (GB)**

(74) Representative: **Gratwick, Christopher et al**
**Imperial Chemical Industries PLC Legal**
**Department: Patents PO Box 6 Bessemer Road**
**Welwyn Garden City Herts, AL7 1HD (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a reactor, in particular a reactor including a catalyst bed with cooling surfaces, and to chemical processes carried out therein.

In such a reactor it has been common to provide the heat exchange surfaces by disposing the catalyst in tubes surrounded by a coolant, for example diphenyl oxide diphenyl mixture (Kotowski, Chemische Technik 1963, *15*, 204—205) or boiling water (GB—A—1205156), and the direction of flow of reactants has been generally parallel to that of the coolant. The possibility of disposing the catalyst outside the tubes and the coolant inside them has been recognised and was used in earlier high pressure ammonia synthesis and methanol synthesis processes, but not in the now standard methanol synthesis at under 300°C, usually at low pressures. Recently a reactor using such a disposition, and also reactants flow transverse to coolant flow, has been proposed (GB—A—2046618) but, by requiring radially-outward or radially-inward flow through the catalyst bed, has presented considerable difficulties, both in mechanical construction and process operation.

It has been proposed in GB—A—1073118 to provide for chordal flow through a catalyst bed, or through a plurality of beds in series. That reference also discloses that, where there are a plurality of such beds, tubes may be disposed in the catalyst free spaces between adjacent beds to effect cooling or heating of the reactants.

According to the invention a vertical-axis cylindrical reactor for exothermic gas-phase catalytic synthesis comprises an outer pressure shell provided with a reactants gas inlet and outlet, and containing, or forming, a vessel for containing a single fixed bed of particulate catalyst, at least one first foraminate chamber within said vessel communicating with said gas inlet and forming the inlet to said catalyst bed, at least one second foraminate chamber within said vessel, communicating with said gas outlet and forming the outlet from said catalyst bed, and tubes for carrying coolant disposed substantially vertically within said catalyst bed and for contact with the particulate catalyst, said foraminate chambers being disposed such that the reactant gas flows from said gas inlet, into said first foraminate chamber or chambers, through said catalyst bed in a mainly chordal direction transverse to the direction of coolant flow, into said second foraminate chamber or chambers and thence to said gas outlet, the total cross sectional area available for reactants flow from said first foraminate chamber or chambers into said catalyst bed being in the range 0.5 to 2 times that available for flow from said catalyst bed into said second foraminate chamber or chambers, and said tubes being disposed such that there are no cooling tubes in the inlet and outlet regions of the catalyst bed, said inlet region extending from the first foraminate chamber or chambers to a distance corresponding to 5—50% of the reactants flow path through said catalyst bed from said first foraminate chamber or chambers to the second foraminate chamber or chambers and said outlet region extending to said second foraminate chamber or chambers from a distance corresponding to 75—95% of the reactants flow path through said catalyst bed from said first foraminate chamber or chambers to said second foraminate chamber or chambers.

The foraminate chambers may be walled-off regions, cages, or spargers. The reactant flow area into the catalyst bed is preferably in the range 0.7 to 1.42 the flow area out of the catalyst bed, and conveniently about equal thereto.

This feature can be arrived at in a number of ways. If the catalyst bed is of annular cross section, the inner wall of the bed can be not much smaller in diameter than the outer wall, but such arrangements limit the length of the flow path through the catalyst. In an annular bed, reactants flow can be in a circumferential direction. However, in the preferred reactor, the catalyst bed inlet or outlet or (preferably) both is provided by a foraminate wall spaced from and intersecting the wall of the catalyst containing vessel. Such a wall can be planar or profiled, for example, can have a radius of curvature greater than that of the vessel. In an alternative reactor the inlet, or outlet, or (preferably) both foraminate chambers, is provided by one or more spargers (possibly in catalyst-free cages) within a single body of catalyst.

The cooling effect of the tubes can be graded according to the differing heat evolution taking place in various parts of the bed. Thus for example where the reaction is very fast the tubes in that region can be more numerous and smaller, and/or finned and/or equipped with internal turbulators to increase the heat transfer coefficient. If desired the coolant can make more than one pass through the bed before leaving it. Another possibility is to use more than one coolant, for example incoming cool reactant or non-boiling liquid in regions of low heat evolution, boiling liquid in regions of high heat evolution. The tubes can if desired be zig-zag in configuration to increase their cooling surface and accommodate thermal expansion.

The catalyst bed is substantially adiabatic, that is, contains no cooling tubes, in its inlet region, i.e. the region extending from the first foraminate chamber, or chambers, to a distance corresponding to 5—50% of the reactants flow path through the catalyst bed from said first foraminate chamber, or chambers, to the second foraminate chamber, or chambers, and in its outlet region, i.e. the region extending to the second foraminate chamber, or chambers, from a distance corresponding to 75—95% of the reactants flow path through the catalyst bed from the first foraminate chamber, or chambers, to the second foraminate chamber, or chambers. This arrangement is particularly suitable for a reactor for methanol synthesis at under 300°C, suitably over a copper-containing catalyst and at a pressure in the range 30—120 bar abs. Such an arrangement affords an inlet zone in which the exothermic heat of synthesis is taken up in raising the synthesis gas temperature to a level at which the reaction rate is optimal (for example 240—270°C), then a region in which the temperature is controlled in that range by the cooling tubes, then a region in which further synthesis takes place to a small extent which is, however, maximised because this region contains only catalyst.

If cooling by boiling liquid is used, this will normally involve a circulatory system in which the boiling liquid is passed to a vapour/liquid separator such as a steam drum and separated liquid is returned to the bottom of the tubes for further heating. The separated liquid passing downwards can also be in tubes within the catalyst bed and may take up heat, without or with boiling, while passing downwards. Alternatively the liquid may give up heat while passing downwards, if the reactants in this region of the bed are at too low a temperature for the intended exothermic catalytic reaction to take place. This is useful if it is desired to limit the preheating of the reactants, or if the catalyst at the inlet region of the bed has lost activity. In a further possibility the liquid can pass downwards through a reactants preheating zone not containing catalyst. The vaporisable liquid flow through the tubes can by thermosiphon action or pumping or both.

One advantage of having coolant tubes within the catalyst bed, so that they are not rigidly fixed to the catalyst containing vessel, is that they can be made of a chromium nickel austenitic stainless steel, such as A.I.S.I. type 304 or 316, which does not significantly react with synthesis gas to give volatile iron carbonyl or nickel carbonyl, but which has a higher thermal expansion coefficient than the low alloy steels preferred for construction of the catalyst containing vessel. Especially if such steels are used, the tubes are connected to the vapour/liquid separator by way of flexible means accommodating expansion.

The following advantages flow from limiting the difference between the inlet and outlet cross sectional areas of the catalyst bed:

(a) since the reactants flow velocities at the inlet and outlet do not differ greatly, provision for a difference in the spacing or heat transfer properties of the cooling tubes is simpler to design and there is no need to introduce localised higher velocity by perforated screens where the area is greater;

(b) the distribution of reactants over the areas of the inlet and outlet can be easily balanced;

(c) if desired, for example to compensate for non-uniform catalyst deactivation, the direction of flow through the catalyst can be reversed.

The reactor is preferably in a combination comprising

(a) a catalyst bed, tubular heat exchange surfaces within the bed, means for feeding an evaporable liquid to the cold side of such surfaces, and means for withdrawing a vapour/liquid mixture from that side; and

(b) a vapour/liquid separator receiving such vapour/liquid mixture, delivering vapour to a user and feeding liquid to the cold side of such heat exchange surfaces.

The vessel containing the catalyst bed and the vessel containing the vapour liquid separator are mounted close together in a common structural framework.

The effect of the close-together mounting in the common structural framework is that losses of pressure in the vapour due to heat losses and tortuous flow paths are minimised, and thus highly efficient heat recovery as medium pressure steam can be achieved. This is especially important when the catalytic reaction (for example methanol synthesis) takes place at under 300°C, so that the steam pressure cannot be much higher than that at which it will be required for synthesis gas generation by steam/hydrocarbon reforming.

Preferably the common structural framework is provided by housing the catalyst bed and the vapour liquid separator within a common external shell. The separator can be accommodated in an upper portion or extension of the shell. The vertical heat exchange tubes in the catalyst bed can communicate with the separator without any passage outside the shell.

The reactor can be of the hot-wall, concrete-lined or cartridge type, depending on the temperature and pressure at which it is to operate.

The invention provides particularly a methanol synthesis process using the reactor. In such a process the pressure is suitably in the range 30 to 120 and more conveniently in the range 40 to 100, bar abs. The temperature is suitably in the range 160—300°C, with a catalyst bed outlet temperature preferably in the range 240—290°C, and lower by 5—30°C at the outlet than at the highest temperature reached. Such temperatures provide for an acceptable methanol output rate (owing to favourable equilibrium) without producing the greater content of impurities that would result from operation at higher temperatures. The methanol output rate is typically in the range 0.3 to 3.0 kg per litre of catalyst per hour. The methanol content of the reacted gas leaving the bed is suitably in the range 3—10% v/v for a process at 50 bar abs. and proportionately more at higher pressures. The volume space velocity through the total catalyst is suitably in the range 5000—50000 hour $^{-1}$. The gas passed over the catalyst is normally a mixture of fresh synthesis gas and unreacted gas recycled from methanol recovery by cooling, condensation and separation, but the process is also suitable for multistage once through operation, using alternating reaction and methanol recovery stages.

The catalyst for methanol synthesis contains copper and usually also zinc oxide and one or more further oxides such as of chromium (UK patent 1010871) or elements from Groups III—IV of the Periodic Table, especially aluminium (UK patent 1159035) or possibly manganese, vanadium, boron and rare earth metals.

The methanol synthesis gas as passed over the catalyst contains hydrogen and carbon monoxide and preferably also, to the extent of 1—20 especially 3—12% v/v, carbon dioxide. The hydrogen content is preferably at least sufficient to convert all the carbon oxides to methanol but may possibly be as little as half this quantity or, more usefully, substantially greater, for example in the range 1.4 to 10 times this quantity. Above-stoichiometric hydrogen contents occur in a recycle process in which the fresh synthesis gas contains more than the stoichiometric quantity of hydrogen, for example when it has been made by steam reforming a hydrocarbon feed-stock containing more than 2 hydrogen atoms per carbon atom or by a process sequence involving carbon dioxide removal. Whichever reactant is in excess in the fresh synthesis gas, its concentration builds up as a result of recycle and is kept down to a design level by purging. In an important form of the invention the purged gas is passed to ammonia synthesis.

The methanol synthesis gas may contain non-reacting gases such as methane, nitrogen or noble gases. Like excess reactants, these also build up during a recycle process and their concentration is kept to a design limit by purging. The gas may contain water vapour preferably sufficient to produce, by the shift reaction, the percentages of carbon dioxide set out above, or to bring the hydrogen to carbon monoxide rates to at least 2.0. Usually the percentage of carbon dioxide, present as such or as the result of shift reaction, is such as to produce a crude methanol containing 5—30% w/w of water.

If desired, the reactor can be used in a so-called "wet" methanol synthesis in which the starting gas contains steam (steam to dry gas ratio 0.1 to 0.3 by volume) in addition to the carbon dioxide already mentioned. Such a process is useful when it is desired to decrease to the maximum extent the carbon monoxide content of the unreacted gas after separation of methanol and water from it, as described in our European published application 11404.

When the reactor is used in a conventional dry methanol synthesis process, it can be followed by a further stage of dry methanol synthesis in a reactor containing an adiabatic bed. This can make possible a useful increase in methanol output in a simple reactor providing a large catalyst volume economically.

In methanol synthesis at 160—300°C the preferred coolant is boiling water and steam is generated at a pressure preferably in the range 20—50 bar abs. The make-up water fed to the cooling tubes is preferably preheated under pressure by heat exchange with reacted synthesis gas leaving the reactor or the subsequent adiabatic bed, if one is used. The steam can be used to drive a turbine or as process steam for synthesis gas generation or to heat water to be used as feed for steam generation or as feed for a humidifier.

Other syntheses to which the invention is applicable include:

(a) dimethyl ether, in which event the catalyst is similar to methanol synthesis catalyst but is associated with a dehydrating agent such as alumina;

(b) higher alcohols, in which event the catalyst may be similar to methanol synthesis catalyst but usually contains alkali metal oxide or manganese oxide or both;

(c) mixed hydrocarbons and/or oxygenated hydrocarbons, in which event catalysts based on iron or cobalt or ruthenium or other metals are used, as in the Fischer-Tropsch, Synthol and other processes;

(d) "OXO" or carbonylation process, for making aldehydes or carboxylic acids, esters or anhydrides, in which the catalyst may be heterogeneous or homogeneous;

(e) methanol synthesis or any of (a) or (d) in combination with a subsequent step of aromatisation, olefinformation over a zeolite or etherification;

(f) methanation of a gas containing a carbon oxide and hydrogen or of a hydrocarbon of higher molecular weight than methane. The starting gas can be a by-product of any of the syntheses (a) to (e) above;

(g) ammonia synthesis; and

(h) ethylene oxide synthesis by reaction of ethylene with oxygen.

One preferred form of the invention is shown in the accompanying drawings, in which

Figure 1 is a sectional elevation;

Figure 2 is a section on the plane indicated by 2 ... 2 in figure 1; and

Figure 3 is a section on a plane through a reactor differing in detail from that shown in figure 1.

In each figure the gas flow direction is indicated by arrows.

The outer shell 10 of the reactor encloses an upper chamber 12 to be described and includes a lower portion 14. Lower portion 14 is divided into three parts by vertical chordal grids (foraminate wall) 16, to enclose catalyst bed region 18 and to provide gas access chambers 20A and 20B, one of which is fed by gas inlet 22 and the other discharges through gas outlet 24. Since 22 and 24 are at opposite ends of chambers 20A and 20B, the length of the path taken by the gas passing through the reactor is approximately the same at all parts of catalyst bed 18. Catalyst bed region 18 is, when the reactor is to be brought into use, charged to level 26 by way of one or more pipes (not shown) extending obliquely inwards from a closable port in outer shell 10. Catalyst can be discharged through closable port 28. Within catalyst bed region is an axial tube 30 leading downwards from upper chamber 12 to primary header 32, which is constituted by a set of horizontal pipes radiating from the bottom of tube 30 but spaced to permit particulate catalyst to pass

7
**0 081 948**
8

between them during charging or discharging. From primary header 32 there project upwardly pipes each leading to secondary headers 34, from each of which extends a set of cooling tubes, each set indicated generally by 36, and terminating in an upper header 38. From each upper secondary header 38 a pipe leads via expansion compensating means such as angles or pigtails to upper chamber *12* beneath baffle 42. Upper chamber *12* is a vapour liquid separator (steam drum) and may contain separating surfaces additional to or instead of baffle 42, following normal practice in the boiler art. Chamber *12* includes steam outlet 44 and its liquid outlet is into tube 30. In order to prevent gas from bypassing the cooled portion of the catalyst bed a set of vertical baffles 40 is disposed between lower secondary headers 34 and between upper headers 38. Such upper baffles 40 extend downwards far enough to be still within bed *18* should catalyst shrinkage occur. As a measure to limit by-passing beneath the cooled portion, this bottom part of bed *18* can be charged with particles, catalytic or not, of smaller size than the catalyst particles in the cooled portion.

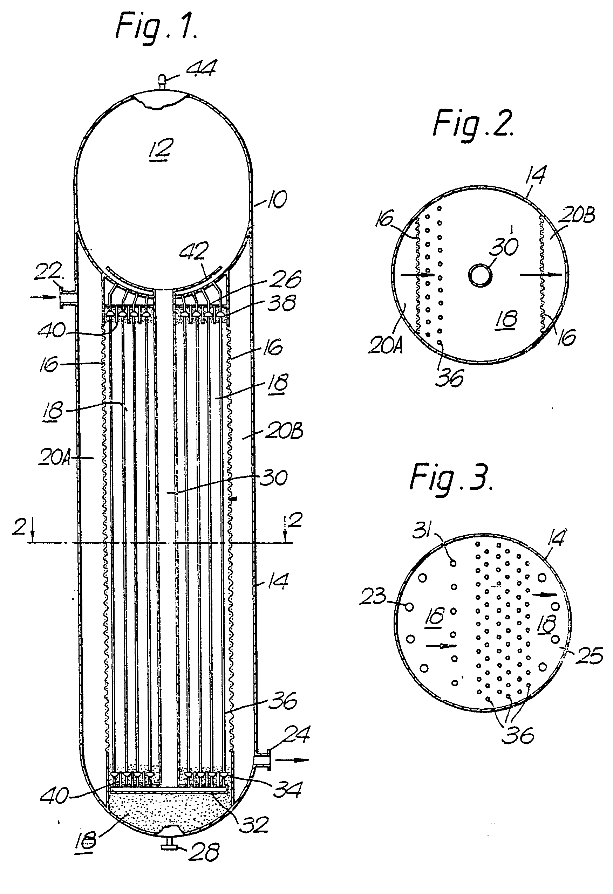

Instead of grids (foraminate wall) 16 and chambers 20A and 20B there may be present inlet and outlet spargers within bed *18* and extending vertically through the full height thereof. This arrangement is shown in plan in figure 3.

If desired, upper chamber *12* can be in a separate shell structurally connected to lower portion 14.

The sectional plan view shown in figure 2 corresponds exactly with figure 1. Of the cooling tubes 36 only two rows have been shown but they are to be assumed to be present throughout catalyst bed 18, or distributed e.g. as in figure 3.

In the reactor represented by figure 3 grids 16 (foraminate wall) are not present, thus permitting the catalyst bed *18* to extend to shell 14. In place of gas upstream access chamber 20A, there are present four inlet spargers 23, which are fed from gas inlet 22 or a similar inlet elsewhere in shell 14. Similarly, in place of gas downstream access chamber 20B there are present four outlet spargers 25, which feed into gas outlet 24 or a similar outlet elsewhere in shell 14. Cooling tubes 36 are disposed asymmetrically so as to present an uncooled relatively large catalyst bed portion to gas that has just entered bed *18* from spargers 23, followed by a cooled portion, followed by a relatively small uncooled portion before the gas leaves by spargers 25. Tubes 36 are present in close sets separated by spaces, to facilitate flow of catalyst during charging and discharging. Instead of axial coolant downward feed tube 30, the connection from the vapour/liquid separator (upper chamber 12) to the headers at the bottom of tubes 36, there are present a plurality (6 shown) of downcomers 31 disposed in a chordal plane in the otherwise uncooled portion of catalyst bed *18*.

Whereas figures 1—3 show a "hot-wall" reactor in which outer shell 14 is pressure-resisting, the reactor can be of the type in which shell 14 is a cartridge made of relatively light gauge metal and supported within an external pressure-resisting shell, so that the pressure of the gas outside the cartridge is approximately equal to that of the gas inside it. This construction may be preferred if the reactor is to be operated with a substantial temperature difference between the inlet and outlet portions of the catalyst bed.

For the methanol synthesis process described above the lengths of gas flow paths in the inlet adiabatic portion, cooled portion and outlet adiabatic portion are in the ratio 40:50:10.

**Claims**

1. A vertical-axis cylindrical reactor for exothermic gas-phase catalytic synthesis comprising an outer pressure shell (10) provided with a reactants gas inlet (22) and outlet (24), and containing, or forming, a vessel for containing a single fixed bed (18) of particulate catalyst, at least one first foraminate chamber (20A; 23) within said vessel communicating with said gas inlet (22) and forming the inlet to said catalyst bed (18), at least one second foraminate chamber (20B; 25) within said vessel, communicating with said gas outlet (24) and forming the outlet from said catalyst bed (18), and tubes (36) for carrying coolant disposed substantially vertically within said catalyst bed and for contact with the particulate catalyst, said foraminate chambers (20A, 20B; 23, 25) being disposed such that the reactant gas flows from said gas inlet (22), into said first foraminate chamber or chambers (20A; 23), through said catalyst bed (18) in a mainly chordal direction transverse to the direction of coolant flow, into said second foraminate chamber or chambers (20B; 25) and thence to said gas outlet (24), the total cross sectional area available for reactants flow from said first foraminate chamber or chambers (20A; 23) into said catalyst bed (18) being in the range 0.5 to 2 times that available for flow from said catalyst bed (18) into said second foraminate chamber or chambers (20B; 25), and said tubes (36) being disposed such that there are no cooling tubes in the inlet and outlet regions of the catalyst bed (18), said inlet region extending from the first foraminate chamber or chambers (20A; 23) to a distance corresponding to 5—50% of the reactants flow path through said catalyst bed (18) from said first foraminate chamber or chambers (20A; 23) to the second foraminate chamber or chambers (20B; 25) and said outlet region extending to said second foraminate chamber or chambers (20B; 25) from a distance corresponding to 75—95% of the reactants flow path through said catalyst bed (18) from said first foraminate chamber or chambers (20A; 23) to said second foraminate chamber or chambers (20B; 25).

2. A reactor according to claim 1 wherein the total cross sectional available for reactants flow from the first foraminate chamber or chambers (20A; 23) into the catalyst bed (18) is in the range 0.7 to 1.42 times that available for flow from said catalyst bed into the second foraminate chamber or chambers (20B; 25).

3. A reactor according to claim 1 or claim 2 wherein at least one of the said first and second foraminate chambers (20A, 20B) is provided by a foraminate wall (grids) (16) spaced from and intersecting the wall (14) of the vessel for containing the catalyst bed (18).

4. A reactor according to claim 1 or claim 2 wherein at least one of said first and second foraminate chambers (23, 25) comprises one or more spargers within the vessel (14) containing the catalyst bed (18).

5. A reactor according to any one of the preceding claims in which the coolant fed through the tubes (36) is a boiling liquid.

6. A reactor according to claim 5 wherein a vapour/liquid separator (12) for receiving a vapour/liquid mixture from said tubes (36), and for separating said mixture, delivering vapour to a user, and returning liquid back to said tubes (36), is provided mounted close to the vessel (14) containing the catalyst and in a common structural framework (10).

7. A reactor according to claim 6 wherein the catalyst bed (18) and the vapour/liquid separator (12) are both housed within said pressure shell (10).

8. A reactor according to any one of the preceding claims in which the coolant tubes (36) are made of chromium nickel austenitic stainless steel.

9. The use of a reactor according to any one of claims 1 to 8 for the synthesis of methanol.

**Patentansprüche**

1. Zylindrischer Reaktor mit vertikaler Hauptachse für exotherme gasphasige katalytische Synthese mit einer äußeren Druckschale (10), die ein Reagenzgaseinlaß (22) und -auslaß (24) aufweist und die enthält oder ausbildet ein Gefäß zur Aufnahme eines einfach befestigten Betts (18) für Feststoff-Katalysator, zumindest eine innerhalb des Gefäßes angeordnete erste gelochtwandige Kammer (20A; 23), die mit dem Gaseinlaß (22) verbunden ist und den Einlaß zum Katalysatorbett (18) bildet, zumindest eine zweite innerhalb des Gefäßes angeordnete gelochtwandige Kammer (20B; 25), die mit dem Gasauslaß (24) verbunden ist und den Auslaß aus dem Katalysatorbett (18) bildet, und Rohre (36) zur Führung von Kühlmitteln, die etwa vertikal innerhalb des Katalysatorbetts angeordnet sind und mit dem Feststoffkatalysator in Berührung sind, wobei die gelochtwandigen Kammern (20A, 20B; 23, 25) derart angeordnet sind, daß das Reagenzgas aus dem Gaseinlaß (22) in die erste gelochtwandige Kammer oder die ersten gelochtwandigen Kammern (20A; 23), durch das Katalysatorbett (18) in etwa Querrichtung senkrecht zur Richtung des Kühlmittelstroms in die zweite gelochtwandige Kammer oder die zweiten gelochtwandigen Kammern (20B; 25) und von dort zum Gasauslaß (25) fließt, wobei die volle Querschnittsfläche, die für den Reagenzfluß von der ersten gelochtwandigen Kammer oder den ersten gelochtwandigen Kammern (20A; 23) in das Katalysatorbett (18) zur Verfügung steht, 0,5 bis 2-fach so groß ist, wie die, die für den Fluß aus dem Katalysatorbett (18) in die zweite gelochtwandige Kammer oder die zweiten gelochtwandigen Kammern (20B; 25) zur Verfügung steht, und wobei die Rohre (36) derart angeordnet sind, daß in den Einlaß- und Auslaßbereichen des Katalysatorbetts (18) keine Kühlrohre angeordnet sind, wobei sich der Einlaßbereich von der ersten gelochtwandigen Kammer oder den ersten gelochtwandigen Kammern (20A, 23) über etwa 5 bis 50% des Reagenzflußwegs durch das Katalysatorbett (18) von der ersten gelochtwandigen Kammer oder den ersten gelochtwandigen Kammern (20A; 23) zu der zweiten gelochtwandigen Kammer oder den zweiten gelochtwandigen Kammern (20B; 25) erstreckt und wobei sich der Auslaßbereich zur zweiten gelochtwandigen Kammer oder zu den zweiten gelochtwandigen Kammern (20B; 25) aus einer Entfernung erstreckt, die sich nach etwa 75 bis 95% des Reagenzflußwegs durch das Katalysatorbett (18) von der ersten gelochtwandigen Kammer oder den ersten gelochtwandigen Kammern (20A; 23) zu der zweiten gelochtwandigen Kammer oder den zweiten gelochtwandigen Kammern (20B, 25) erstreckt.

2. Reaktor nach Anspruch 1, bei dem die totale Querschnittsfläche, die für den Reagenzfluß aus der ersten gelochtwandigen oder den ersten gelochtwandigen Kammern (20A; 23) in das Katalysatorbett (18) zur Verfügung steht, etwa 0,7 bis 1,42-fach so groß ist, wie die, die zum Fluß aus dem Katalysatorbett in die zweite gelochtwandige Kammer oder die zweiten gelochtwandigen Kammern (20B, 25) zur Verfügung steht.

3. Reaktor nach Anspruch 1 oder 2, bei dem zumindest eine der ersten und zweiten gelochtwandigen Kammern (20A, 20B) durch eine gelochte Wand (Gitter) (16) geschaffen ist, die von der Gefäßwand (14) beabstandet ist und diese schneidet, so daß sie das Katalysatorbett (18) aufnimmt.

4. Reaktor nach Anspruch 1 oder 2, bei dem zumindest eine der ersten und zweiten gelochtwandigen Kammern (23, 25) einen oder mehrere Zerstäuber innerhalb des Gefäßes (14) aufweist, das das Katalysatorbett (18) enthält.

5. Reaktor nach einem der vorangehenden Ansprüche, bei dem das durch die Rohre (36) geführt Kühlmedium eine Siedeflüssigkeit ist.

6. Reaktor nach Anspruch 5, bei dem ein Dampf/Flüssigkeit-Separator (12) zur Aufnahme einer Dampf/Flüssigkeit-Mischung aus den Rohren (36) und zur Trennung der Mischung, zur Abgabe von Dampf an einen Verbraucher und zur Rückgabe der Flüssigkeit zurück zu den Rohren (36) vorgesehen ist, der nahe am Gefäß (14) montiert ist, das den Katalysator enthält, und der mit diesem in einem gemeinsamen Gefügerahmen (10) angeordnet ist.

7. Reaktor nach Anspruch 6, bei dem das Katalysatorbett (18) und der Dampf/Flüssigkeits-Separator (12) innerhalb der Druckschale (10) angeordnet sind.

8. Reaktor nach einem der vorangehenden Ansprüche, bei dem die Kühlrohre (36) aus austenitischem rostfreiem Chromnickelstahl hergestellt sind.

9. Anwendung eines Reaktor gemäß einem der Patentansprüche 1 bis 8 zur Methanolsynthese.

**Revendications**

1. Réacteur cylindrique à axe vertical pour synthèse catalytique exothermique en phase gazeuse, comprenant une enveloppe extérieure (10) résistant à la pression qui comporte une entrée (22) et une sortie (24) deréactifs gazeux, et contenant, ou formant, une cuve pour contenir un lit fixe unique (18) de catalyseur particulier, au moíns une première chambre à paroi perforée (20A; 23) agencée à l'intérieur de ladite cuve de manière à communiquer avec ladite entrée de gaz (22) et à constituer l'entrée audit lit de catalyseur (18), au moins une deuxième chambre à paroi perforée (20B; 25) agencée à l'intérieur de ladite cuve de manière à communiquer avec ladite sortie de gaz (24) et à constituer la sortie dudit lit de catalyseur (18), et des tubes (36) pour véhiculer un fluide de refroidissement disposés sensiblement verticalement à l'intérieur du lit de catalyseur et en contact avec le catalyseur particulier, les dites chambres à paroi perforée (20A, 20B; 23, 25) étant disposées de sorte que le réactif gazeux circule à partir de ladite entrée de gaz (22), pénètre dans ladite première chambre, ou les premières chambres, à paroi perforée (20A; 23), traverse le lit de catalyseur (18) dans une direction principalement suivant une corde et transversalement à la direction de circulation de fluide de refroidissement, pénètre dans ladite deuxième chambre, ou les deuxièmes chambres, à paroi perforée (20B; 25), et arrive ensuite à ladite sortie de gaz (24), la section totale disponible pour l'écoulement des réactifs gazeux de ladite première chambre, ou des premières chambres, à paroi perforée (20A; 23) audit lit de catalyseur (18) étant de l'ordre de 0,5 à 2 fois celle qui est disponible pour l'écoulement dudit lit de catalyseur (18) à ladite deuxième chambre, ou aux deuxièmes chambres, à paroi perforée (20B, 25), et lesdits tubes (36) étant disposés de sorte qu'il n'y a pas de tubes de refroidissement dans les régions d'entrée et de sortie du lit de catalyseur (18), ladite région d'entrée s'étendant à partir de ladite première chambre, ou des premières chambres, à paroi perforée (20A; 23) jusqu'à une distance correspondant à 5—50% du parcours des réactifs à travers ledit lit de catalyseur (18) entre ladite première chambre, ou les premières chambres, à paroi perforée (20A; 23) et ladite deuxième chambre, ou les deuxièmes chambres, à paroi perforée (20B; 25) et ladite région de sortie s'étendant jusqu'à ladite deuxième chambre, ou les deuxièmes chambres, à paroi perforée (20B; 25) à partir d'une distance correspondant à 75—95% du parcours des réactifs à travers ledit lit de catalyseur (18) entre ladite première chambre, ou les premières chambres, à paroi perforée (20A; 23) et ladite deuxième chambre, ou les deuxièmes chambres, à paroi perforée (20B; 25).

2. Réacteur suivant la revendication 1, dans lequel la section totale disponible pour l'écoulement des réactifs de la première chambre, ou des premières chambres, à paroi perforée (20A; 23) au lit de catalyseur (18) est de l'ordre de 0,7 à 1,42 fois celle qui est disponible pour l'écoulement dudit lit de catalyseur à la deuxième chambre, ou aux deuxièmes chambres, à paroi perforée (20B; 25).

3. Réacteur suivant la revendication 1 ou la revendication 2, dans lequel au moins l'une desdites première et deuxième chambres à paroi perforée (20A, 20B) est définie par une paroi perforée (grilles) (16), espacée de la paroi (14) de la cuve contenant le lit de catalyseur (18) et en intersection avec celle-ci.

4. Réacteur suivant la revendication 1 ou la revendication 2, dans lequel au moins l'une desdites première et deuxième chambres à paroi perforée (23, 25) est constituée par un ou plusieurs tubes distributeurs disposés à l'intérieur de la cuve (14) contenant le lit de catalyseur (18).

5. Réacteur suivant l'une quelconque des revendications précédentes, dans lequel le fluide de refroidissement qui circule dans les tubes (36) est un liquide bouillant.

6. Réacteur suivant la revendication 5, dans lequel un séparateur vapeur/liquide (12), pour recevoir un mélange vapeur/liquide venant desdits tubes (36) et pour séparer ce mélange, diriger la vapeur vers un utilisateur et renvoyer le liquide vers lesdits tubes (36), est monté près de la cuve (14) contenant le catalyseur et dans une structure commune (10).

7. Réacteur suivant la revendication 6, dans lequel le lit de catalyseur (18) et le séparateur vapeur/liquide (12) sont tous deux logés à l'intérieur de ladite enveloppe (10) résistant à la pression.

8. Réacteur suivant l'une quelconque des revendications précédentes, dans lequel les tubes (36) de fluide de refroidissement sont fabriqués en acier inoxydable austénitique au nickel-chrome.

9. Utilisation d'un réacteur suivant l'une quelconque des revendications 1 à 8 pour la synthèse du méthanol.

Fig.1.

Fig.2.

Fig.3.